# EUROPÄISCHE PATENTSCHRIFT

(11) **EP 3 574 834 B1**
(45) Veröffentlichungstag und Bekanntmachung des Hinweises auf die Patenterteilung: **13.05.2020**
(21) Anmeldenummer: 18175101.7
(22) Anmeldetag: 30.05.2018
(51) Int. Cl.: A61B 6/00, G21K 1/02, G21K 1/10

(54) **STREUSTRAHLENRASTER FÜR EINE MEDIZINISCHE RÖNTGENEINRICHTUNG**
ANTI-SCATTER GRID FOR A MEDICAL X-RAY DEVICE
GRILLE ANTI-DIFFUSION POUR UN APPAREIL À RAYONS X MÉDICAL

(43) Veröffentlichungstag der Anmeldung: 04.12.2019
(73) Patentinhaber: Siemens Healthcare GmbH, 91052 Erlangen (DE)
(72) Erfinder: Korkmaz, Hatice, 91058 Erlangen (DE); Strattner, Peter, 91560 Heilsbronn (DE)

(56) Entgegenhaltungen:
- CN-U- 205 286 379
- GB-A- 439 728
- US-A1- 2006 115 052
- US-A1- 2007 147 587
- US-B1- 6 327 341

## Beschreibung

Die Erfindung betrifft ein Streustrahlenraster für eine medizinische Röntgeneinrichtung und die Röntgeneinrichtung.

Ein Streustrahlenraster wird typischerweise bei einer medizinischen Bildgebung mit Röntgenstrahlen verwendet, wobei üblicherweise hohe Anforderungen an eine Bildqualität gestellt werden. Bei der medizinischen Bildgebung wird typischerweise ein zu untersuchendes Objekt, beispielsweise ein Patient, von den Röntgenstrahlen durchleuchtet, welche insbesondere von einer Röntgenröhre emittiert werden. Eine Schwächungsverteilung der Röntgenstrahlen auf der der Röntgenröhre gegenüberliegenden Seite des Patienten wird typischerweise mittels eines Röntgendetektors zweidimensional erfasst. Alternativ oder zusätzlich kann eine zeilenweise Erfassung der durch den Patienten geschwächten Röntgenstrahlen mittels einer derartigen Röntgeneinrichtung erfolgen, welche für eine Computertomographie ausgebildet ist.

Jeder Bildpunkt einer Röntgenaufnahme der medizinischen Bildgebung entspricht vorzugsweise einem Schwächungswert der Röntgenstrahlen durch den Patienten auf einer geradlinigen Achse von der Röntgenröhre zu einem dem Bildpunkt entsprechenden Ort auf dem Röntgendetektor. Die Röntgenröhre weist typischerweise eine punktförmige Röntgenquelle auf. Diejenigen Röntgenstrahlen, welche von der punktförmigen Röntgenquelle auf dieser Achse geradlinig auf dem Röntgendetektor auftreffen, werden Primärstrahlen genannt. Typischerweise werden einige von der Röntgenröhre emittierten Röntgenstrahlen bei einer Durchleuchtung des Patienten gestreut, so dass zusätzlich zu den Primärstrahlen Streustrahlen, welche Sekundärstrahlen genannt werden können, auf dem Röntgendetektor auftreffen.

Von dem Röntgendetektor werden typischerweise bis zu 10-mal mehr Streustrahlen als Primärstrahlen erfasst, wenn für die medizinische Bildgebung kein Streustrahlenraster verwendet wird. Die Streustrahlung weist üblicherweise allerdings keine Bildinformation auf. Aufgrund der Streustrahlung können eine Bildqualität und/oder eine Bildauflösung der medizinischen Bildgebung verringert sein.

Das Streustrahlenraster, wenn es für die medizinische Bildgebung verwendet wird, absorbiert typischerweise einen Teil der Streustrahlung, wodurch insbesondere die Bildqualität und/oder die Bildauflösung der medizinischen Bildgebung verbessert werden kann. Das Streustrahlenraster weist üblicherweise regelmäßig angeordnete Röntgenstrahlen stark absorbierende und Röntgenstrahlen schwach absorbierende Strukturen auf. Ein herkömmliches Streustrahlenraster ist typischerweise in Linientechnik hergestellt.

Aus der DE 103 54 811 B4 ist ein weiteres Streustrahlenraster, insbesondere für medizinische Röntgeneinrichtungen, bekannt, das sich aus zahlreichen durch ein Füll- und Trägermaterial voneinander getrennten Absorptionselementen für Röntgenstrahlung zusammensetzt, die annähernd parallel zueinander oder auf einen gemeinsamen Fokus hin ausgerichtet und zufällig verteilt angeordnet sind, dadurch gekennzeichnet, dass die Absorptionselemente einzelne Fasern aus einem Röntgenstrahlung stark absorbierenden Material sind und dass das Füll- und Trägermaterial durch einzelne Fasern aus einem für Röntgenstrahlung weitgehend transparentem Material gebildet ist.

Die CN 205 286 379 U offenbart ein Streustrahlenraster mit einer Vielzahl von radial beginnenden, spiralförmigen und ineinander verschachtelten Absorptionslamellen.

Die DE 10 2012 217 965 A1 beschreibt ein fokussierbares Streustrahlenraster für Röntgenstrahlung mit abwechselnd gestapelten ersten und zweiten folienartigen Streifen.

GB439728 A offenbart einen Streustrahlenraster für eine medizinische Röntgeneinrichtung, aufweisend eine Absorptionslamelle, welche für eine Absorption von Röntgenstrahlen der medizinischen Röntgeneinrichtung wobei die Absorptionslamelle eine Spirale bildet und wobei die Spirale einen zentralen Bereich aufweist.

Der Erfindung liegt die Aufgabe zu Grunde, ein Streustrahlenraster für eine medizinische Röntgeneinrichtung und die Röntgeneinrichtung anzugeben, mit welchen eine höhere Bildqualität bei der medizinischen Bildgebung erreicht wird.

Die Aufgabe wird durch die Merkmale der unabhängigen Ansprüche gelöst. Vorteilhafte Ausgestaltungen sind in den Unteransprüchen beschrieben.

Das erfindungsgemäße Streustrahlenraster für eine medizinische Röntgeneinrichtung weist
- eine Absorptionslamelle, welche für eine Absorption von Röntgenstrahlen der medizinischen Röntgeneinrichtung ausgebildet ist, und
- zumindest einen Absorptionsstift auf, welcher für eine Absorption der Röntgenstrahlen der medizinischen Röntgeneinrichtung ausgebildet ist,
- wobei die Absorptionslamelle eine Spirale bildet und
- wobei die Spirale einen zentralen Bereich aufweist, in welchem der zumindest eine Absorptionsstift angeordnet ist.

Das erfindungsgemäße Streustrahlenraster bietet insbesondere folgende Vorteile:
Vorteilhafterweise ist eine Streustrahlung bei einer medizinischen Bildgebung, für welche das Streustrahlenraster verwendet wird, verringert, wodurch typischerweise eine Bildqualität und/oder eine Bildauflösung erhöht ist. Das Streustrahlenraster kann vorzugsweise eine Reduzierung einer Röntgendosis, welche typischerweise mit einer Energie und/oder einer Anzahl der Röntgenstrahlen korreliert, ermöglichen.

Der zentrale Bereich ist vorzugsweise derart ausgestaltet, dass eine Durchstrahlungscharakteristik des zentralen Bereichs und eine Durchstrahlungscharakteristik eines Bereichs außerhalb vom zentralen Bereich sich entsprechen. Das bedeutet insbesondere, dass vorzugsweise im zentralen Bereich weniger, besonders vorzugsweise keine Inhomogenitäten auftreten. In anderen Worten werden bei einer Durchleuchtung die Röntgenstrahlen vorzugsweise gleichmäßig absorbiert. Aufgrund der dadurch verbesserten Homogenität sind vorzugsweise die Bildqualität und/oder die Bildauflösung höher als bei einem herkömmlichen Streustrahlenraster.

Vorteilhafterweise kann das Streustrahlenraster mit der Absorptionslamelle und dem zumindest einen Absorptionsstift einfacher, beispielsweise im Vergleich zu einem herkömmlichen Streustrahlenraster mit Linientechnik, hergestellt werden. Besonders vorteilhafterweise kann das Streustrahlenraster, insbesondere die Absorptionslamelle, kostengünstig hergestellt werden.

Ein weiterer Vorteil kann sein, dass aufgrund einer Kombination des zumindest einen Absorptionsstifts und der Absorptionslamelle, welche die Spirale bildet, das Streustrahlenraster für verschiedene bildgebende Untersuchungen, insbesondere bei einer Radiographie und/oder Fluoroskopie, verwendet werden kann.

Vorteilhafterweise ist eine Sichtbarkeit des Streustrahlenrasters auf einem Röntgendetektor verringert, besonders vorteilhafterweise eliminiert, insbesondere wenn das Streustrahlenraster eine Drehbewegung bei der medizinischen Bildgebung durchführt. Aufgrund der Drehbewegung und/oder aufgrund der Kombination der Absorptionslamelle mit dem zumindest einen Absorptionsstift kann das Streustrahlenraster vergleichsweise gröbere Linien, insbesondere eine gröbere Absorptionslamelle, aufweisen, um an eine Pixelmatrix des Röntgendetektors angepasst zu werden. Das bedingt eine wesentliche Vereinfachung der Herstellung und eine Reduzierung der Kosten, insbesondere wenn die Pixelmatrix sehr hochauflösend ist und/oder die Bildauflösung sehr hoch ist.

Ein weiterer Vorteil des Streustrahlenrasters kann sein, dass typischerweise das Streustrahlenraster desto günstiger herzustellen ist, je größer die Absorptionslamelle im Vergleich zu dem zumindest einen Absorptionsstift ist. In anderen Worten können mittels der Absorptionslamelle die Streustrahlen in einem beliebig großen Bereich vorzugsweise kostengünstiger absorbiert werden als mit dem zumindest einen Absorptionsstift. Typischerweise ist also die Absorptionslamelle günstiger herzustellen als der zumindest eine Absorptionsstift.

Eine Ausführungsform sieht vor, dass das Streustrahlenraster lediglich die Absorptionslamelle aufweist. Diese Ausführungsform ist insofern vorteilhaft, weil dadurch das Streustrahlenraster einfach aufzubauen ist. In anderen Worten weist diese Ausführungsform vorzugsweise lediglich eine einzige Absorptionslamelle auf. Insbesondere im Vergleich zu einem herkömmlichen Streustrahlenraster mit einer Vielzahl an spiralförmigen Absorptionslamellen gemäß der CN 205 286 379 U ist es vorteilhaft, dass lediglich die einzige Absorptionslamelle in dem Streustrahlenraster angeordnet, insbesondere aufgewickelt, ist. Denn das herkömmliche Streustrahlenraster mit der Vielzahl an spiralförmigen Absorptionslamellen weist insbesondere einen Nachteil auf, nämlich dass eine Herstellung sehr aufwändig ist, weil die Absorptionslamellen sich jeweils kreuzen können und/oder ineinander verschachtelt sind.

Eine Ausführungsform sieht vor, dass jeder Absorptionsstift in dem zentralen Bereich angeordnet ist. Dadurch kann das Streustrahlenraster vorteilhafterweise in einer kurzen Herstellungszeit aufgebaut werden, weil beispielsweise die Absorptionslamelle um den zumindest einen Absorptionsstift herumgewickelt wird. Weiterhin ist diese Ausführungsform vorteilhaft, weil das Streustrahlenraster modular aufgebaut sein kann.

Eine Ausführungsform sieht vor, dass die Spirale eine archimedische Spirale ist. Ein Vorteil dieser Ausführungsform ist insbesondere, dass die archimedische Spirale typischerweise einen konstanten Windungsabstand aufweist. Vorteilhafterweise weist ein Bereich des Streustrahlenrasters, welcher die archimedische Spirale aufweist, vorzugsweise eine Primärstrahlentransparenz auf, welche über den gesamten Bereich hinweg homogen ist.

Eine Ausführungsform sieht vor, dass jeder Absorptionsstift in dem zentralen Bereich angeordnet ist und dass die Spirale eine archimedische Spirale ist. Diese Ausführungsform bietet insbesondere einen Vorteil, dass die Primärstrahlentransparenz des Streustrahlenrasters vorzugsweise in jedem Bereich des Streustrahlenrasters homogen ist.

Eine Ausführungsform sieht vor, dass die Spirale in dem zentralen Bereich einen geschlossenen Ring bildet, wobei jeder Absorptionsstift in dem Ring angeordnet ist. Dadurch ist vorzugsweise eine einfache und damit kostengünstige Herstellung des Streustrahlenrasters möglich, weil die Spirale um die Absorptionsstifte herum gewickelt werden kann.

Eine Ausführungsform sieht vor, dass das Streustrahlenraster weniger als 100 Absorptionsstifte aufweist. Diese Ausführungsform bietet insbesondere einen Vorteil, dass aufgrund der Anzahl an Absorptionsstiften das Streustrahlenraster kostengünstig hergestellt werden kann und/oder eine homogene Durchstrahlungscharakteristik aufweist.

Eine Ausführungsform sieht vor, dass das Streustrahlenraster mehr als 4 und weniger als 51 Absorptionsstifte aufweist. Diese Ausführungsform ist besonders vorteilhaft, weil die Spirale einfach um die Absorptionsstifte herum gewickelt werden kann. Besonders vorteilhaft ist diese Ausführungsform, wenn die Spirale in dem zentralen Bereich einen geschlossenen Ring bildet, wobei jeder Absorptionsstift in dem Ring angeordnet ist.

Eine Ausführungsform sieht vor, dass die Absorptionslamelle Blei aufweist und der zumindest eine Absorptionsstift Wolfram und/oder Tantal aufweist. Ein Vorteil dieser Ausführungsform kann sein, dass der zumindest eine Absorptionsstift aus einem anderen Material als die Absorptionslamelle aufgebaut sein kann. Denn beispielsweise weisen Wolfram und/oder Tantal eine höhere Festigkeit und/oder einen höheren Einkaufspreis im Vergleich zu Blei auf. Diese Ausführungsform bietet daher insbesondere einen Vorteil, dass für das Streustrahlenraster eine hohe Flexibilität bei einer Auswahl der Materialien für die Absorptionslamelle und den zumindest einen Absorptionsstift besteht.

Eine Ausführungsform sieht vor, dass das Streustrahlenraster eine Längsachse durch den zentralen Bereich aufweist, wobei die Absorptionslamelle eine erste Strecke entlang der Längsachse abdeckt, wobei der zumindest eine Absorptionsstift eine zweite Strecke entlang der Längsachse abdeckt und wobei ein Anteil der zweiten Strecke an einer Gesamtstrecke aus der ersten Strecke und der zweiten Strecke geringer ist als 5%. Diese Ausführungsform bietet insbesondere einen Vorteil, dass relativ zu einer Gesamtfläche des Streustrahlenrasters der zumindest eine Absorptionsstift üblicherweise eine kleinere Fläche als die Absorptionslamelle abdeckt, wodurch das Streustrahlenraster typischerweise einfacher und/oder kostengünstiger aufgebaut werden kann.

Eine Ausführungsform sieht vor, dass der Anteil der zweiten Strecke an der Gesamtstrecke geringer ist als 1%. Dadurch ist vorzugsweise ein besonders kostengünstiges Streustrahlenraster herstellbar, weil die Absorptionslamelle im Wesentlichen die Gesamtfläche des Streustrahlenrasters abdeckt.

Eine Ausführungsform sieht vor, dass eine Durchstrahlungscharakteristik des zumindest einen Absorptionsstifts an eine Durchstrahlungscharakteristik der Absorptionslamelle durch Variation zumindest eines Parameters der folgenden Liste angepasst ist:
- Abstand um den zumindest einen Absorptionsstift herum,
- Durchmesser des zumindest einen Absorptionsstifts,
- Länge des zumindest einen Absorptionsstifts,
- Material des zumindest einen Absorptionsstifts. Die Durchstrahlungscharakteristik des zumindest einen Absorptionsstifts und die Durchstrahlungscharakteristik der Absorptionslamelle sind vorzugsweise derart aufeinander abgestimmt, dass die Absorption der Röntgenstrahlen gleichmäßig erfolgt. In anderen Worten sind vorteilhafterweise die Primärstrahlentransparenz des zumindest einen Absorptionsstifts und die Primärstrahlentransparenz der Absorptionslamelle gleich. Diese Ausführungsform bietet insbesondere einen weiteren Vorteil, weil mehrere Möglichkeiten zur Anpassung der Durchstrahlungscharakteristik bestehen, wodurch das Streustrahlenraster optimal aufgebaut sein kann. Des Weiteren ist vorzugsweise der zumindest eine Absorptionsstift derart angepasst, insbesondere dimensioniert, dass eine Sichtbarkeit des zumindest einen Absorptionsstifts auf dem Röntgendetektor verringert ist.

Die erfindungsgemäße Röntgeneinrichtung weist das Streustrahlenraster auf. Die Röntgeneinrichtung bietet insbesondere einen Vorteil, weil eine hohe Bildqualität und/oder eine hohe Bildauflösung bei der medizinischen Bildgebung erreicht werden kann.

Bei der Beschreibung der Vorrichtung erwähnte Merkmale, Vorteile oder alternative Ausführungsformen sind ebenso auf das Verfahren zu übertragen und umgekehrt. Mit anderen Worten können Ansprüche auf das Verfahren mit Merkmalen der Vorrichtung weitergebildet sein und umgekehrt. Insbesondere kann die erfindungsgemäße Vorrichtung in dem Verfahren verwendet werden.

Im Folgenden wird die Erfindung anhand der in den Figuren dargestellten Ausführungsbeispiele näher beschrieben und erläutert. Grundsätzlich werden in der folgenden Figurenbeschreibung im Wesentlichen gleich bleibende Strukturen und Einheiten mit demselben Bezugszeichen wie beim erstmaligen Auftreten der jeweiligen Struktur oder Einheit benannt.

Es zeigen:
Fig. 1 ein Streustrahlenraster in einem ersten Ausführungsbeispiel in einer Vogelperspektive,
Fig. 2 ein Streustrahlenraster des ersten Ausführungsbeispiels in einer perspektivischen Ansicht,
Fig. 3 ein Streustrahlenraster in einem zweiten Ausführungsbeispiel,
Fig. 4 ein Streustrahlenraster in einem dritten Ausführungsbeispiel,
Fig. 5 zumindest einen Absorptionsstift in einem vierten Ausführungsbeispiel und
Fig. 6 eine Röntgeneinrichtung in einem fünften Ausführungsbeispiel.

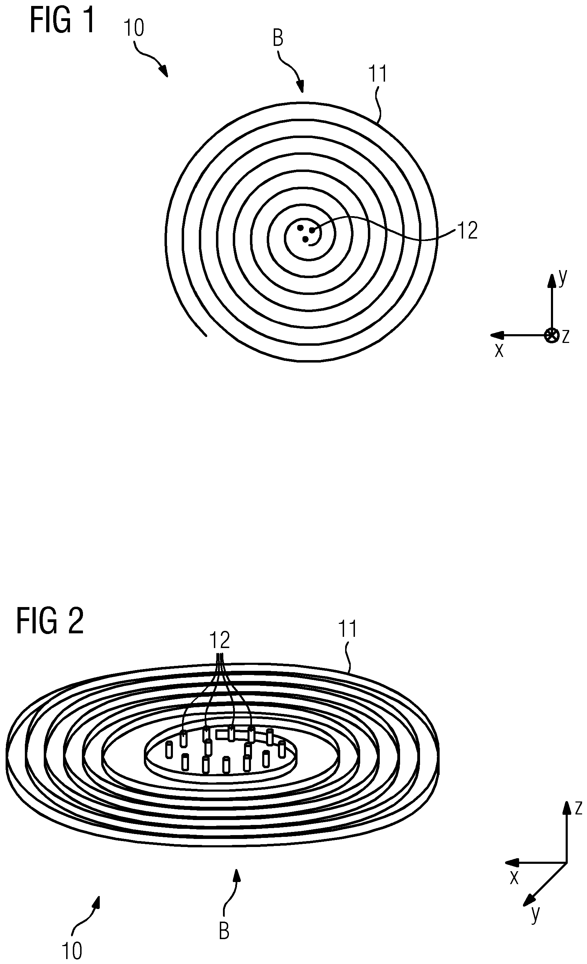

**Fig. 1** zeigt ein Streustrahlenraster 10 für eine medizinische Röntgeneinrichtung 20 aus einer Vogelperspektive. Das Streustrahlenraster 10 weist eine Absorptionslamelle 11 und zumindest einen Absorptionsstift 12 auf. Die Absorptionslamelle 11 ist für eine Absorption von Röntgenstrahlen der medizinischen Röntgeneinrichtung 20 ausgebildet. Der zumindest eine Absorptionsstift 12 ist für eine Absorption der Röntgenstrahlen der medizinischen Röntgeneinrichtung 20 ausgebildet. Die Absorptionslamelle 11 und/oder der zumindest eine Absorptionsstift 12 absorbieren beispielsweise diejenigen Röntgenstrahlen, welche auf der Absorptionslamelle 11 bzw. dem zumindest einen Absorptionsstift 12 auftreffen. Diese Röntgenstrahlen werden typischerweise Streustrahlung genannt. Vorzugsweise weist das Streustrahlenraster 10 Bereiche auf, durch welche die Röntgenstrahlen hindurch propagieren können, wobei diese Röntgenstrahlen typischerweise Primärstrahlen genannt werden. In anderen Worten besteht typischerweise zumindest eine Sichtverbindung zwischen einer Röntgenröhre 21 und einem Röntgendetektor 22.

In diesem Ausführungsbeispiel ist das Streustrahlenraster 10 relativ zu der Röntgenröhre 21 derart ausgerichtet, dass die Röntgenstrahlen, welche von der Röntgenröhre 21 emittiert werden, im Zentralstrahl typischerweise senkrecht auf dem Streustrahlenraster 10 auftreffen. Die Röntgenstrahlen werden beispielsweise in der medizinischen Röntgeneinrichtung 20 für eine medizinische Bildgebung mittels Durchleuchtung verwendet.

Die Absorptionslamelle 11 bildet eine Spirale. Die Absorptionslamelle 11 kann daher spiralförmig und/oder schneckenförmig sein. Die Spirale weist einen zentralen Bereich B auf, in welchem der zumindest eine Absorptionsstift 12 angeordnet ist. Die Spirale weist üblicherweise mehrere Windungen auf. In anderen Worten wird die Absorptionslamelle 11 beispielsweise mehrmals um einen Mittelpunkt des Streustrahlenrasters 10, insbesondere um einen Wickelstift, herumgewickelt. Der Wickelstift kann nach dem Wickeln der Absorptionslamelle 11 aus dem Streustrahlenraster 10 herausgezogen werden. Alternativ kann der Wickelstift dem zumindest einen Absorptionsstift 12 entsprechen, welcher typischerweise in dem Streustrahlenraster 10 verbleibt. Eine schneckenförmige Spirale weist typischerweise variierende Abstände zwischen den Windungen auf. Das Streustrahlenraster 10 weist beispielsweise eine Ausdehnung entlang einer y-Achse und/oder entlang einer x-Achse zwischen 10 cm und 100 cm, besonders vorzugsweise zwischen 23 cm und 43 cm auf. Typischerweise ist eine Ausdehnung des Streustrahlenrasters 10, insbesondere der Spirale, entlang einer x-Achse und/oder y-Achse desto größer, je mehr Windungen die Spirale aufweist.

Grundsätzlich ist es denkbar ein aufgrund der Spirale im Wesentlichen kreisförmiges Streustrahlenraster 10 für die medizinische Bildgebung zu verwenden. Alternativ kann ein eckiges Streustrahlenraster aus dem kreisförmigen Streustrahlenraster 10 herausgeschnitten und/oder herausgesägt werden, welches für die medizinische Bildgebung verwendet werden kann. Insbesondere ist es denkbar, dass ein weiterer Absorptionsstift außerhalb der Spirale angeordnet ist, um dadurch das eckige Streustrahlenraster zu bilden. Des Weiteren ist es denkbar, dass der weitere Absorptionsstift für eine robuste Ausführung des Streustrahlenrasters 10 an einer beliebigen Windung der Spirale angeordnet ist. Das eckige Streustrahlenraster ist typischerweise viereckig, insbesondere rechteckig oder quadratisch.

Der zumindest eine Absorptionsstift 12 ist in Fig. 1 punktförmig abgebildet.

**Fig. 2** zeigt das Streustrahlenraster 10 in einer perspektivischen Ansicht.

Der zumindest eine Absorptionsstift 12 weist typischerweise eine zylindrische Form auf und/oder ist faserförmig. Der zumindest eine Absorptionsstift 12, insbesondere eine Längsachse des zumindest einen Absorptionsstifts 12, ist parallel zu einer z-Achse ausgerichtet. Eine Länge 12.L des zumindest einen Absorptionsstifts 12 entlang der z-Achse beträgt beispielsweise 1 bis 20 mm. Eine Ausdehnung der Absorptionslamelle 11 entlang der z-Achse beträgt beispielsweise 1 bis 20 mm. Die Länge des zumindest einen Absorptionsstift 12 und die Ausdehnung der Absorptionslamelle 11 entlang der z-Achse weisen typischerweise eine gleiche Größenordnung auf. Ein Durchmesser 12.D des zumindest einen Absorptionsstifts 12 ist beispielsweise kleiner als 1 mm, besonders vorteilhafterweise kleiner als 100 µm und größer als 20 µm.

Die Absorptionslamelle 11 kann beispielsweise aus einem Materialverbund bestehen. Der Materialverbund kann ein die Röntgenstrahlen absorbierendes Material und ein für die Röntgenstrahlen im Wesentlichen transparentes Material aufweisen. Beispielsweise kann der Materialverbund ein kaschiertes Blei-Papier-Band aufweisen. Die Absorptionslamelle 11 kann vorzugsweise von einem Band für die Herstellung des Streustrahlenrasters 10 aufgewickelt werden. Die Absorptionslamelle 11 weist typischerweise eine bandförmige Struktur auf. Die Absorptionslamelle 11 ist typischerweise länger als breit und breiter als tief, wenn die Absorptionslamelle 11 in einer abgerollten Form vorliegt. In anderen Worten ist üblicherweise die Ausdehnung in Längsrichtung innerhalb der x-y-Ebene zumindest eine Größenordnung größer als die Ausdehnung parallel zur z-Achse. Die Absorptionslamelle 11 ist typischerweise aus einem Stück aufgebaut. In anderen Worten bildet die Absorptionslamelle 11 typischerweise ein einziges Absorptionselement, welches insbesondere die Spirale bildet. Die Spirale liegt in diesem Ausführungsbeispiel in der x-y-Ebene.

Das die Röntgenstrahlen absorbierende Material kann insbesondere Blei, Wolfram, Tantal und/oder Legierungen aus Blei, Wolfram und/oder Tantal aufweisen. Typischerweise absorbieren Blei, Wolfram, Tantal und/oder die Legierungen aus Blei, Wolfram und/oder Tantal die Röntgenstrahlen stark. Der zumindest eine Absorptionsstift 12 besteht typischerweise aus dem die Röntgenstrahlen absorbierenden Material. Das für die Röntgenstrahlen im Wesentlichen transparente Material weist beispielsweise Kunststoff, Schaumstoff, Papier und/oder Fiber auf und/oder kann beispielsweise mittels eines physikalischen und/oder chemischen Prozesses vorzugsweise im Anschluss an die Wicklung aus der Absorptionslamelle 11 herausgelöst werden.

Das Streustrahlenraster 10 kann beispielsweise derart hergestellt werden, dass der zumindest eine Absorptionsstift 12 mittels einer Schussvorrichtung durch eine mit einem Kleber benetzte Schicht geschossen wird. Beispielsweise kann der zumindest eine Absorptionsstift 12 dadurch in dem zentralen Bereich B der Spirale befestigt werden.

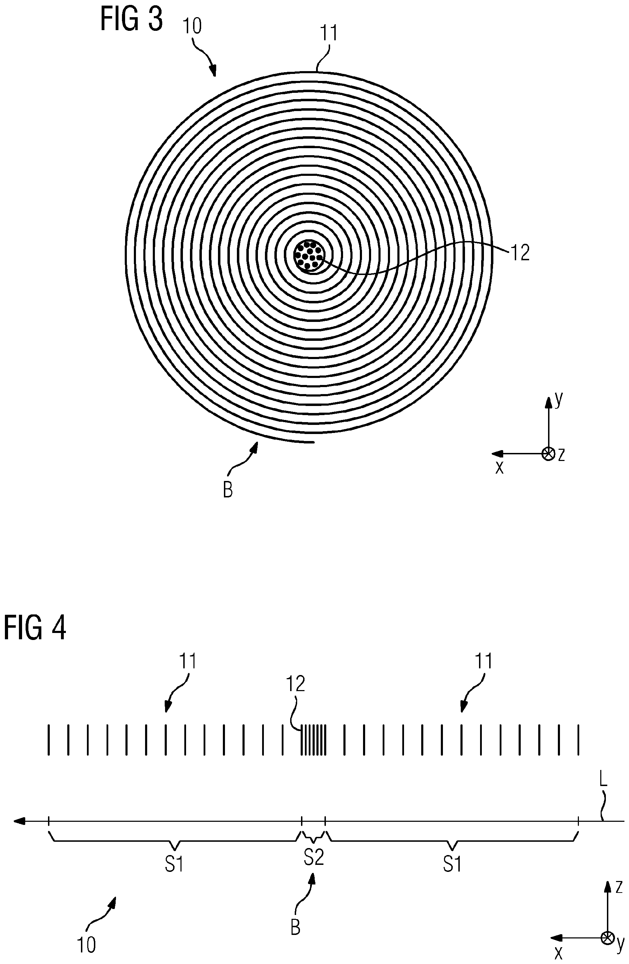

**Fig. 3** zeigt das Streustrahlenraster 10 in einem zweiten Ausführungsbeispiel.

Jeder Absorptionsstift 12 ist in dem zentralen Bereich B angeordnet. In anderen Worten ist jeder Absorptionsstift 12 in einem Mittenbereich des Streustrahlenrasters 10 angeordnet. Die Absorptionslamelle 11 ist also um den zumindest einen Absorptionsstift 12 herum angeordnet.

Die Spirale ist eine archimedische Spirale. In anderen Worten ist ein Radius r der Spirale proportional zu einem Drehwinkel ϕ:
r = a · ϕ, wobei a > 0.

Die Spirale bildet in dem zentralen Bereich B einen geschlossenen Ring, wobei jeder Absorptionsstift 12 in dem Ring angeordnet ist. Der geschlossene Ring kann beispielsweise den zentralen Bereich B vollständig eingrenzen und/oder definieren. Ein erstes Ende der Absorptionslamelle 11 beginnt typischerweise nicht in einem mathematischen Nullpunkt der Spirale, sondern berührt die Absorptionslamelle 11 an einem Punkt, um dadurch einen geschlossenen Ring zu bilden. Typischerweise kann die Spirale die archimedische Spirale ab dem Berührungspunkt bilden. In anderen Worten bilden typischerweise diejenigen Windungen außerhalb des geschlossenen Rings die archimedische Spirale.

Grundsätzlich ist es denkbar, dass die Absorptionslamelle 11 einen ersten Bereich, welche für die Absorption der Röntgenstrahlen ausgebildet ist und beispielsweise das die Röntgenstrahlen absorbierende Material aufweist, und einen zweiten Bereich aufweist, wobei der zweite Bereich vorzugsweise an dem ersten Ende der Absorptionslamelle 11 den geschlossenen Ring bildet und das für die Röntgenstrahlen im Wesentlichen transparente Material aufweist.

Das Streustrahlenraster 10 weist weniger als 100 Absorptionsstifte auf. In diesem Ausführungsbeispiel weist das Streustrahlenraster 10 mehr als 4 und weniger als 51 Absorptionsstifte auf.

In diesem Ausführungsbeispiel weist die Absorptionslamelle 11 Blei auf. Der zumindest eine Absorptionsstift 12 weist Wolfram und/oder Tantal auf. Grundsätzlich ist es denkbar, dass die Absorptionslamelle 11 und/oder der zumindest eine Absorptionsstift 12 Legierungen aus Blei und/oder Wolfram und/oder Tantal aufweist. Wenn das Streustrahlenraster 10 einen ersten Absorptionsstift und einen zweiten Absorptionsstift aufweist, kann der erste Absorptionsstift Wolfram und/oder der zweite Absorptionsstift Tantal aufweisen. In anderen Worten können der erste Absorptionsstift und der zweite Absorptionsstift und/oder die Absorptionslamelle 11 verschiedene die Röntgenstrahlen absorbierende Materialien aufweisen.

Das Streustrahlenraster 10 weist lediglich die Absorptionslamelle 11 auf. In anderen Worten weist das Streustrahlenraster 10 keine weiteren Absorptionslamellen zusätzlich zu der Absorptionslamelle 11 auf, sondern lediglich die Absorptionslamelle 11. Die Absorptionslamelle 11 ist vorzugsweise nicht überkreuzt angeordnet.

**Fig. 4** zeigt das Streustrahlenraster 10 in einem dritten Ausführungsbeispiel in einem Querschnitt. Das Streustrahlenraster 10, insbesondere die Absorptionslamelle 11 und/oder der zumindest eine Absorptionsstift 12, kann parallel oder fokussiert ausgebildet sein. In diesem Ausführungsbeispiel ist das Streustrahlenraster 10 parallel ausgebildet. In anderen Worten sind in diesem Fall die Windungen parallel zueinander ausgerichtet. Die Absorptionslamelle 11 kann vorzugsweise bei einer Herstellung derart fokussiert werden, indem beispielsweise eine Kleberaupe und/oder eine Kunststoffraupe an der Rückseite der Absorptionslamelle 11, insbesondere in einem Zwischenraum zwischen den Windungen, angebracht wird.

Das Streustrahlenraster 10 weist eine Längsachse L durch den zentralen Bereich B auf, wobei die Längsachse L in Fig. 4 aus Gründen der Übersichtlichkeit unterhalb des Streustrahlenrasters 10 abgebildet ist. Die Längsachse L ist in diesem Fall parallel zur x-Achse ausgerichtet. Das Streustrahlenraster 10 ist in der x-y-Ebene ausgerichtet.

Die Absorptionslamelle 11 deckt eine erste Strecke S1 entlang der Längsachse L ab und der zumindest eine Absorptionsstift 12 deckt eine zweite Strecke S2 entlang der Längsachse L ab. Ein Anteil der zweiten Strecke S2 an einer Gesamtstrecke aus der ersten Strecke S1 und der zweiten Strecke S2 ist geringer als 5%. In anderen Worten wird die Gesamtstrecke durch ein Zusammenfügen der ersten Strecke S1 und der zweiten Strecke S2 gebildet. Die Gesamtstrecke entspricht typischerweise einer Ausdehnung des Streustrahlenrasters 10 entlang der Längsachse L. Die Gesamtstrecke kann einem Durchmesser der Absorptionslamelle 11 entsprechen. In anderen Worten deckt die Absorptionslamelle 11 entlang der Längsachse L mindestens 95% ab. Die Längsachse L geht insbesondere durch einen Mittelpunkt des Streustrahlenrasters 10.

In einem weiteren Ausführungsbeispiel ist der Anteil der zweiten Strecke an der Gesamtstrecke geringer als 1%.

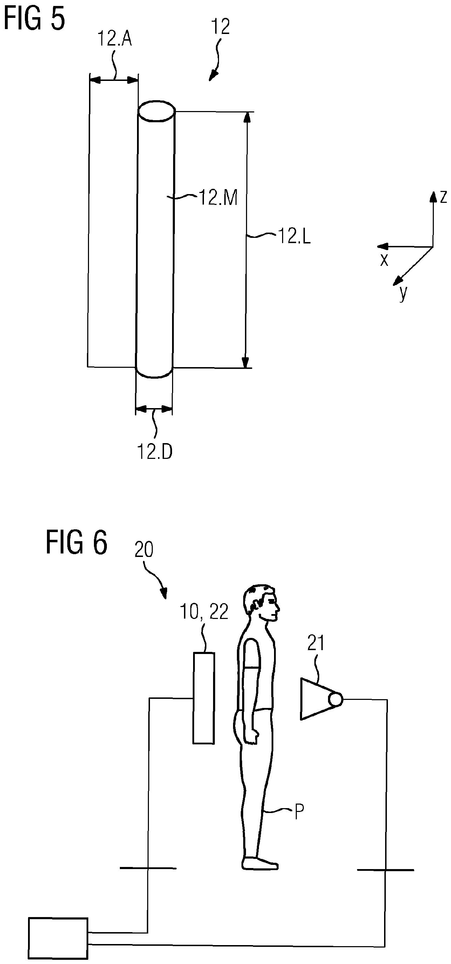

**Fig. 5** zeigt den zumindest einen Absorptionsstift 12 in einem vierten Ausführungsbeispiel in einer Detailansicht.

Eine Durchstrahlungscharakteristik des zumindest einen Absorptionsstifts 12 ist an eine Durchstrahlungscharakteristik der Absorptionslamelle 11 durch Variation zumindest eines Parameters der folgenden Liste angepasst:
- Abstand 12.A um den zumindest einen Absorptionsstift 12 herum,
- Durchmesser 12.D des zumindest einen Absorptionsstifts 12,
- Länge 12.L des zumindest einen Absorptionsstifts 12,
- Material 12.M des zumindest einen Absorptionsstifts 12.

Die Durchstrahlungscharakteristik kann eine Primärstrahlentransparenz und/oder eine Streustrahlentransparenz beschreiben. Die Absorption der Streustrahlung durch die Absorptionslamelle 11 und/oder dessen Absorption durch den zumindest einen Absorptionsstift 12 hängen üblicherweise von der Durchstrahlungscharakteristik ab. Beispielsweise ist die Primärstrahlentransparenz desto höher, je weniger Röntgenstrahlen, insbesondere Primärstrahlen, die Absorptionslamelle 11 und/oder der zumindest eine Absorptionsstift 12 absorbieren. Die Streustrahlentransparenz ist üblicherweise desto niedriger, je mehr Röntgenstrahlen, insbesondere Streustrahlen, die Absorptionslamelle 11 und/oder der zumindest eine Absorptionsstift 12 absorbieren. Vorzugsweise führt das Streustrahlenraster 10 zu einer hohen Primärstrahlentransparenz und einer geringen Streustrahlentransparenz.

Der Abstand 12.A ist typischerweise ein Luftraum um den zumindest einen Absorptionsstift 12 herum und/oder weist vorzugsweise Luft auf. Der Luftraum kann alternativ beispielsweise aus dem für die Röntgenstrahlen im Wesentlichen transparenten Material ausgefüllt sein. Die Röntgenstrahlen gehen typischerweise durch den Luftraum hindurch.

Das Material 12.M kann beispielsweise dem die Röntgenstrahlen absorbierenden Material entsprechen.

**Fig. 6** zeigt die Röntgeneinrichtung 20 in einem fünften Ausführungsbeispiel. Die Röntgeneinrichtung 20 weist ein Streustrahlenraster 10 auf. In diesem Ausführungsbeispiel weist die Röntgeneinrichtung 20 zusätzlich die Röntgenröhre 21 und einen Röntgendetektor 22 auf. Der Röntgendetektor 22 kann mehrere Detektorelemente aufweisen. Das Streustrahlenraster 10 ist typischerweise relativ zu der Röntgenröhre 21 und dem Röntgendetektor 22 ausgerichtet. Die Röntgeneinrichtung 20 kann zu einer medizinischen Bildgebung, insbesondere zu einer konventionellen Röntgenbildgebung, zu einer Radiographie und/oder zu einer Fluoroskopie, ausgebildet sein. Grundsätzlich ist es denkbar, dass die Röntgeneinrichtung 20 für eine Angiographie und/oder eine Computertomographie ausgebildet ist.

Die Röntgenstrahlen werden typischerweise von der Röntgenröhre 21 erzeugt und emittiert. Bei der medizinischen Bildgebung durchdringen die Röntgenstrahlen typischerweise einen Patienten P, wobei die Röntgenstrahlen absorbiert, abgeschwächt und/oder gestreut werden. Vor dem Röntgendetektor 22 ist vorzugsweise das Streustrahlenraster 10 angeordnet, um die Streustrahlung, vorzugsweise zu einem großen Teil, zu absorbieren.

Das Streustrahlenraster 10 kann beispielsweise während der medizinischen Bildgebung translatorisch und/oder rotatorisch bewegbar sein.

Obwohl die Erfindung im Detail durch die bevorzugten Ausführungsbeispiele näher illustriert und beschrieben wurde, so ist die Erfindung dennoch nicht durch die offenbarten Beispiele eingeschränkt.

## Patentansprüche

1. Streustrahlenraster (10) für eine medizinische Röntgeneinrichtung (20), aufweisend
- eine Absorptionslamelle (11), welche für eine Absorption von Röntgenstrahlen der medizinischen Röntgeneinrichtung (20) ausgebildet ist, und
- zumindest einen Absorptionsstift (12), welcher für eine Absorption der Röntgenstrahlen der medizinischen Röntgeneinrichtung (20) ausgebildet ist,
- wobei die Absorptionslamelle (11) eine Spirale bildet und
- wobei die Spirale einen zentralen Bereich (B) aufweist, in welchem der zumindest eine Absorptionsstift (12) angeordnet ist.

2. Streustrahlenraster (10) nach Anspruch 1, wobei jeder Absorptionsstift (12) in dem zentralen Bereich (B) angeordnet ist.

3. Streustrahlenraster (10) nach einem der vorhergehenden Ansprüche, wobei die Spirale eine archimedische Spirale ist.

4. Streustrahlenraster (10) nach den Ansprüchen 2 und 3.

5. Streustrahlenraster (10) nach einem der vorhergehenden Ansprüche, wobei die Spirale in dem zentralen Bereich (B) einen geschlossenen Ring bildet, wobei jeder Absorptionsstift (12) in dem Ring angeordnet ist.

6. Streustrahlenraster (10) nach einem der vorhergehenden Ansprüche, wobei das Streustrahlenraster (10) weniger als 100 Absorptionsstifte (12) aufweist.

7. Streustrahlenraster (10) nach Anspruch 6, wobei das Streustrahlenraster (10) mehr als 4 und weniger als 51 Absorptionsstifte (12) aufweist.

8. Streustrahlenraster (10) nach einem der vorhergehenden Ansprüche, wobei die Absorptionslamelle (11) Blei aufweist und der zumindest eine Absorptionsstift (12) Wolfram und/oder Tantal aufweist.

9. Streustrahlenraster (10) nach einem der vorhergehenden Ansprüche, wobei das Streustrahlenraster (10) eine Längsachse (L) durch den zentralen Bereich (B) aufweist, wobei die Absorptionslamelle (11) eine erste Strecke (S1) entlang der Längsachse (L) abdeckt, wobei der zumindest eine Absorptionsstift (12) eine zweite Strecke (S2) entlang der Längsachse (L) abdeckt und wobei ein Anteil der zweiten Strecke (S2) an einer Gesamtstrecke aus der ersten Strecke (S1) und der zweiten Strecke (S2) geringer ist als 5%.

10. Streustrahlenraster (10) nach Anspruch 9, wobei der Anteil der zweiten Strecke (S2) an der Gesamtstrecke geringer ist als 1%.

11. Streustrahlenraster (10) nach einem der vorhergehenden Ansprüche, wobei eine Durchstrahlungscharakteristik des zumindest einen Absorptionsstifts (12) an eine Durchstrahlungscharakteristik der Absorptionslamelle (11) durch Variation zumindest eines Parameters der folgenden Liste angepasst ist:
- Abstand (12.A) um den zumindest einen Absorptionsstift (12) herum,
- Durchmesser (12.D) des zumindest einen Absorptionsstifts (12),
- Länge (12.L) des zumindest einen Absorptionsstifts (12),
- Material (12.M) des zumindest einen Absorptionsstifts (12).

12. Streustrahlenraster (10) nach einem der vorhergehenden Ansprüche, wobei das Streustrahlenraster (10) lediglich die Absorptionslamelle (11) aufweist.

13. Röntgeneinrichtung (20), aufweisend ein Streustrahlenraster (10) nach einem der vorhergehenden Ansprüche.

## Claims

1. Anti-scatter grid (10) for a medical X-ray device (20), comprising
- an absorption lamella (11) which is designed for absorption of X-rays from the medical X-ray device (20), and
- at least one absorption pin (12) which is used for absorption of the X-rays from the medical X-ray device (20),
- wherein the absorption lamella (11) forms a spiral and
- wherein the spiral has a central region (B) in which the at least one absorption pin (12) is arranged.

2. Anti-scatter grid (10) according to claim 1, wherein each absorption pin (12) is arranged in the central region (B).

3. Anti-scatter grid (10) according to one of the preceding claims, wherein the spiral is an Archimedean spiral.

4. Anti-scatter grid (10) according to claims 2 and 3.

5. Anti-scatter grid (10) according to one of the preceding claims, wherein the spiral forms a closed ring in the central region (B), wherein each absorption pin (12) is arranged in the ring.

6. Anti-scatter grid (10) according to one of the preceding claims, wherein the anti-scatter grid (10) has fewer than 100 absorption pins (12).

7. Anti-scatter grid (10) according to claim 6, wherein the anti-scatter grid (10) has more than 4 and fewer than 51 absorption pins (12).

8. Anti-scatter grid (10) according to one of the preceding claims, wherein the absorption lamella (11) comprises lead and the at least one absorption pin (12) comprises tungsten and/or tantalum.

9. Anti-scatter grid (10) according to one of the preceding claims, wherein the anti-scatter grid (10) has a longitudinal axis (L) through the central region (B), wherein the absorption lamella (11) covers a first segment (S1) along the longitudinal axis (L), wherein the at least one absorption pin (12) covers a second segment (S2) along the longitudinal axis (L) and wherein the percentage of the second segment (S2) in a total segment comprising the first segment (S1) and the second segment (S2) is less than 5%.

10. Anti-scatter grid (10) according to claim 9, wherein the percentage of the second segment (S2) in the total segment is less than 1%.

11. Anti-scatter grid (10) according to one of the preceding claims, wherein an irradiation characteristic of the at least one absorption pin (12) is adapted to an irradiation characteristic of the absorption lamella (11) by varying at least one parameter from the following list:
- distance (12.A) around the at least one absorption pin (12),
- diameter (12.D) of the at least one absorption pin (12),
- length (12.L) of the at least one absorption pin (12),
- material (12.M) of the at least one absorption pin (12).

12. Anti-scatter grid (10) according to one of the preceding claims, wherein the anti-scatter grid (10) merely comprises the absorption lamella (11).

13. X-ray device (20), comprising an anti-scatter grid (10) according to one of the preceding claims.

## Revendications

1. Grille (10) anti-diffusion d'un appareil (20) médical à rayons X, comportant
- une lamelle (11) d'absorption, constituée pour une absorption de rayons X du dispositif (20) médical à rayons X, et
- au moins un crayon (12) d'absorption, constitué pour absorber les rayons X du dispositif (20) médical à rayons X,
- dans laquelle la lamelle (11) d'absorption forme une spirale et
- dans laquelle la spirale a une partie (B) centrale, dans laquelle est disposé le au moins un crayon (12) d'absorption.

2. Grille (10) anti-diffusion suivant la revendication 1, dans laquelle chaque crayon (12) d'absorption est disposé dans la partie (B) centrale.

3. Grille (10) anti-diffusion suivant l'une des revendications précédentes, dans laquelle la spirale est une spirale d'Archimède.

4. Grille (10) anti-diffusion suivant les revendications 2 et 3.

5. Grille (10) anti-diffusion suivant l'une des revendications précédente, dans laquelle la spirale forme, dans la partie (B) centrale, un anneau fermé, dans laquelle chaque crayon (12) d'absorption est disposé dans l'anneau.

6. Grille (10) anti-diffusion suivant l'une des revendications précédentes, dans laquelle la grille (10) anti-diffusion a moins de 100 crayons (12) d'absorption.

7. Grille (10) anti-diffusion suivant la revendication 6, dans laquelle la grille (10) anti-diffusion a plus de 4 et moins de 51 crayons (12) d'absorption.

8. Grille (10) anti-diffusion suivant l'une des revendications précédentes, dans laquelle la lamelle (11) d'absorption a du plomb et le au moins un crayon (12) d'absorption a du tungstène et/ou du tantale.

9. Grille (10) anti-diffusion suivant l'une des revendications précédentes, dans laquelle la grille (10) anti-diffusion a un axe (L) longitudinal passant par la partie (B) centrale, dans laquelle la lamelle (11) d'absorption recouvre une première étendue (S1) le long de l'axe (L) longitudinal, dans laquelle le au moins un crayon (12) d'absorption recouvre une deuxième étendue (S2) le long de l'axe (L) longitudinal et dans laquelle une proportion de la deuxième étendue (S2), par rapport à l'étendue totale composée de la première étendue (S1) et de la deuxième étendue (S2), est plus petite que 5%.

10. Grille (10) anti-diffusion suivant la revendication 9, dans laquelle la proportion de la deuxième étendue (S2), par rapport à l'étendue totale, est plus petite que 1%.

11. Grille (10) anti-diffusion suivant l'une des revendications précédentes, dans laquelle une caractéristique de rayonnement du au moins un crayon (12) d'absorption est adaptée à une caractéristique de rayonnement de la lamelle (11) d'absorption par variation d'un paramètre de la liste suivante :
- distance (12.A) autour du au moins un crayon (12) d'absorption,
- diamètre (12.D) du au moins un crayon (12) d'absorption,
- longueur (12.L) du au moins un crayon (12) d'absorption,
- matériau (12.M) du au moins un crayon (12) d'absorption.

12. Grille (10) anti-diffusion suivant l'une des revendications précédentes, dans laquelle la grille (10) anti-diffusion a seulement la lamelle (11) d'absorption.

13. Dispositif (20) à rayons X, ayant une grille (10) anti-diffusion suivant l'une des revendications précédentes.
